(19) Europäisches Patentamt

European Patent Office

Office européen des brevets

(11) **EP 0 986 325 B1**

(12) **EUROPÄISCHE PATENTSCHRIFT**

(45) Veröffentlichungstag und Bekanntmachung des Hinweises auf die Patenterteilung:
**15.09.2004 Patentblatt 2004/38**

(51) Int Cl.⁷: **A61B 5/03**, G01L 11/00

(21) Anmeldenummer: **98934876.8**

(22) Anmeldetag: **05.06.1998**

(86) Internationale Anmeldenummer:
**PCT/DE1998/001563**

(87) Internationale Veröffentlichungsnummer:
**WO 1998/056292 (17.12.1998 Gazette 1998/50)**

(54) **SENSORSYSTEM IN FORM EINES KATHETERS ZUR MESSUNG VON DRUCKPROFILEN**

CATHETER-SHAPED SENSOR SYSTEM FOR MEASURING PRESSURIZED SECTIONS

SYSTEME DE DETECTION SOUS FORME DE CATHETER POUR LA MESURE DE CARACTERISTIQUES MANOMETRIQUES

(84) Benannte Vertragsstaaten:
**AT BE CH DE DK ES FI FR GB GR IE IT LI LU MC NL PT SE**

(30) Priorität: **07.06.1997 DE 19724001**

(43) Veröffentlichungstag der Anmeldung:
**22.03.2000 Patentblatt 2000/12**

(73) Patentinhaber: **PP-Technologies AG**
**3186 Düdingen (CH)**

(72) Erfinder: **Knoll, Meinhard**
**48565 Steinfurt (DE)**

(74) Vertreter: **Besse, François**
**ANDRE ROLAND IPS,**
**15, Avenue Tissot,**
**P.O. Box 1255**
**1001 Lausanne (CH)**

(56) Entgegenhaltungen:
EP-A- 0 632 992          DE-A- 2 117 541
US-A- 4 476 880          US-A- 4 873 990

**Beschreibung**

Die Erfindung betrifft ein Sensorsystem zur Messung von Druckprofilen. Solche Systeme lassen sich überall dort einsetzen, wo ein- oder zweidimensionale Druckverteilungen gemessen werden sollen. Ein wichtiges Einsatzfeld ist die Medizin. Hier lassen sich Druckprofilmessungen zum Beispiel in der Urologie, der Proktologie, der Kardiologie und anderen Disziplinen mit Hilfe von Kathetern durchführen.

Es ist bekannt, daß Sensoren zur Messung von Drücken eingesetzt werden. Solche Sensoren sind so klein, daß sie sich z.B. in Katheter einbauen lassen (vgl. z.B.: R.L. Smith, S.D. Collins: Sensor Design and Packaging, in W. Göpel, J. Hesse, J.N. Zemel: Sensors, Vol. 1, VCH Verlagsgesellschaft, Weinheim, 1989, pp. 79-106).

Nachteilig bei solchen Sensoren ist, daß mit ihrer Hilfe nur Drücke an einem Ort gemessen werden können. Zur Aufnahme von Druckprofilen müßten Drucksensor-Arrays eingesetzt werden, oder der Sensor müßte während der Messung bewegt werden.

Sensor-Arrays sind technisch aufwendig und teuer; die Bewegung von Sensoren ist in vielen Anwendungsfällen nur schwer durchführbar oder gar nicht möglich.

Die DE-OS 21 17 541 offenbart eine Sonde zur Messung des Druckes in Körpergeweben, die zwei voneinander getrennte mit einer Flüssigkeit gefüllte und miteinander kommunizierende Kompartimente aufweist, wobei eines der Kompartimente ausdehnbar in einen starren Mantel eingeschlossen und das andere Kompartiment durch den äußeren Druck kompressibel ist. Beide Kompartimente bilden einen elektrischen Spannungsteiler für die in der eingefüllten Flüssigkeit über jedem der Kompartimente abfallenden Spannung, über den der äußere Druck erfaßt werden kann.

Die EP 0 632 992 offenbart einen Drucksensor aus einem kompressiblen Hohlkörper an dessen innerer Oberfläche zwei Elektroden angeordnet sind und dessen Hohlraum mit Kohlepartikeln gefüllt ist. In Abhängigkeit vom äußeren Druck ändert sich dabei der Widerstand der Kohlefüllung zwischen den beiden Elektroden.

Der Erfindung liegt darum die Aufgabe zugrunde, ein Meßsystem zu realisieren, bei dem ein Druckprofil gemessen werden kann, ohne daß der Katheter dabei bewegt werden muß. Auf den Einsatz aufwendiger Sensor-Arrays soll dabei verzichtet werden.

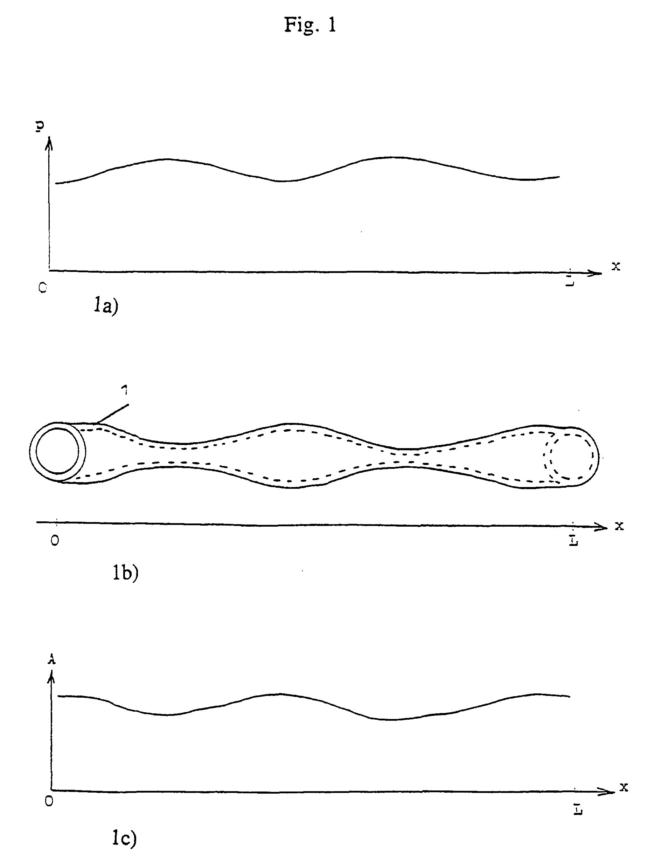

Diese Aufgabe wird erfindungsgemäß dadurch gelöst, daß der Meßkatheter als schlauchartiger flexibler Hohlkörper (1) der Länge L - nachfolgend "Schlauch" genannt - ausgebildet ist (Fig. 1b). Der Schlauch hat einen inneren kreisrunden, ovalen, rechteckigen oder anders geformten Querschnitt der Größe A. Eine äußere Druckbelastung p(x) (Fig. 1a) wird am Schlauch (1) als eine Querschnittsfunktion A(x) (Fig. 1c) abgebildet. Dies erfolgt durch ein Zusammenpressen des Schlauches (1), das durch die Differenz von Außenund Innendruck verursacht wird.

Der Zusammenhang zwischen der äußeren Druckbelastung p(x) und der Querschnittsfunktion A(x) kann im Sinne einer Meßbereichseinstellung durch eine Veränderung des im Inneren des Schlauches (1) vorgegebenen Drucks po variiert werden.

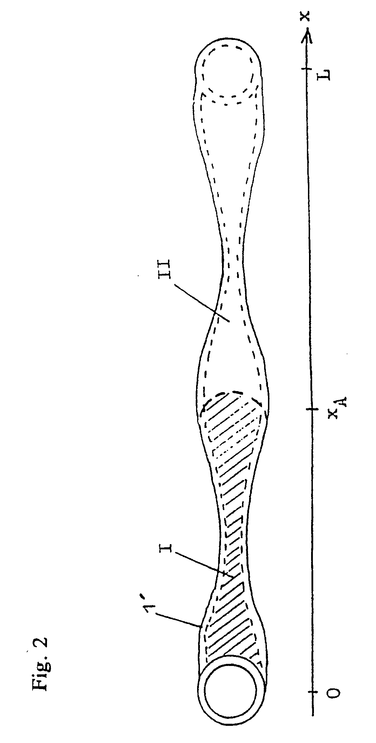

Die Abtastung des ortsabhängigen Querschnittes A(x) geschieht dadurch, daß der Schlauch von einer Seite her bei (x=0) kontinuierlich mit einem flüssigen Stoff (I) befüllt wird, der den Stoff (II) bis zur Fülllänge $x_A$ verdrängt (Fig. 2).

Für den Zusammenhang zwischen der Fülllänge $x_A$ und dem Füllvolumen $V_f$ gilt

$$dx_A = \frac{dV_f}{A(x_A)}$$

Daraus folgt das Füllvolumen $V_f$ (Stoff I):

$$V_f = \int_{x=0}^{x=x_A} A(x)\,dx$$

Die Messung der Fülllänge $x_A$ kann nach unterschiedlichen Methoden erfolgen:

- Messung des elektrischen Widerstandes
- Messung der elektrischen Kapazität
- Messung der akustischen Hohlraumresonanz.

Auf einfache Weise kann die Erfassung der Fülllänge $x_A$ durch Messung des elektrischen Widerstandes mit Hilfe eines Meßgerätes (4) erfolgen.

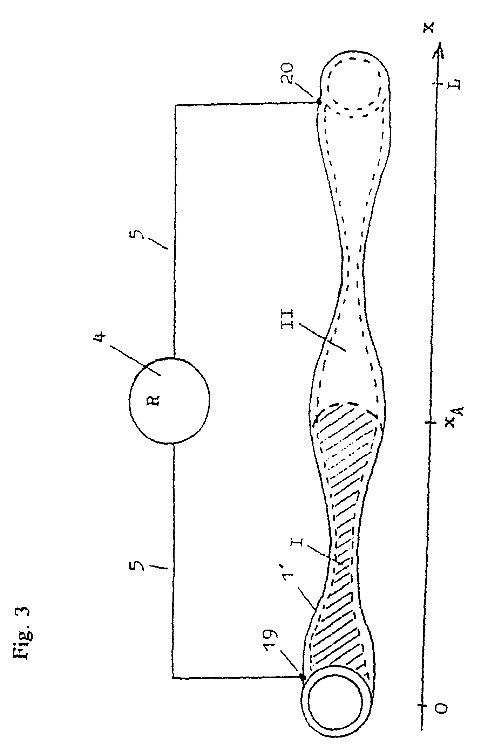

Bei einem der möglichen Meßverfahren wird das Meßgerät (4) über die elektrischen Zuleitungen (5) mit dem Schlauch (1') verbunden (Fig. 3). Die Messung kann mit Gleichstrom oder Wechselstrom bei elektrischen Spannungen von U > 10 mV erfolgen.

Der Schlauch (1') mit der Länge L hat einen elektrischen Widerstand im Bereich von $10^2$ bis $10^7$ Ω.

Dies wird dadurch erreicht, daß der Schlauch (1') aus elektrisch leitendem Kunststoff hergestellt ist, oder die innere Oberfläche mit einem elektrisch leitenden Film überzogen ist.

Zur Befüllung des Schlauches (1') wird ein Stoff (I) mit einem spezifischen Widerstand $\rho_1$ verwendet. Der verdrängte Stoff (II) hat einen spezifischen Widerstand $\rho_2$. Es muß gelten:

$$\rho_2 \gg \rho_1$$

Stoff (I) kann jede elektrisch leitfähige Flüssigkeit (z.B. Salzlösung: NaCl, KCl...) sein. Stoff (II) kann Luft, ein anderes Gas, aber auch eine Flüssigkeit (z.B. Öl) sein, die die Bedingung $\rho_2 \gg \rho_1$ erfüllt.

Ist pro Längeneinheit entlang des Schlauches der Widerstand des Stoffes (I) sehr viel geringer als der Widerstand des Schlauches, so wird in dem mit Stoff (I) befüllten Bereich der elektrische Widerstand des Schlauches annähernd kurzgeschlossen.

Bei Befüllung des Schlauches ergibt sich somit ein meßbarer elektrischer Widerstand R als Funktion des Füllvolumens. Da die Füllänge $x_A$ im Bereich kleiner Querschnitte A schneller zunimmt als in Bereichen größerer Querschnitte, ergeben sich im Verlauf der Funktion R $(V_f)$ in den Bereichen mit kleinen Querschnitten größere Beträge der Steigung. Der Differentialquotient $dR/dV_f$ ist damit ein Abbild der Querschnittsfunktion A $(X_A)$ :

$$\frac{dR}{dV_f} \sim \left( -\frac{1}{A(x_A)} \right)$$

Für den meßbaren Widerstand R ergibt sich folgender Ansatz:

$$R = \frac{R_f}{L} \cdot x_A + \frac{R_s}{L} (L - x_A)$$

Hierin ist $R_f$ der elektrische Widerstand des gefüllten Schlauches, $R_s$ der Widerstand des leeren Schlauches und L die Schlauchlänge.

Für $R_f \ll R_s$ ergibt sich für den Verlauf des elektrischen Widerstandes R als Funktion der Füllänge $x_A$

$$R_s \approx -\frac{1}{L} x_A + 1$$

und hieraus für $x_A$

$$x_A \approx L \left( 1 - \frac{R}{R_s} \right)$$

Der Verlauf des Druckprofiles p(x) wird durch den Betrag des Differentialquotienten $dR/dV_f$ über der Füllänge $x_A$ abgebildet.

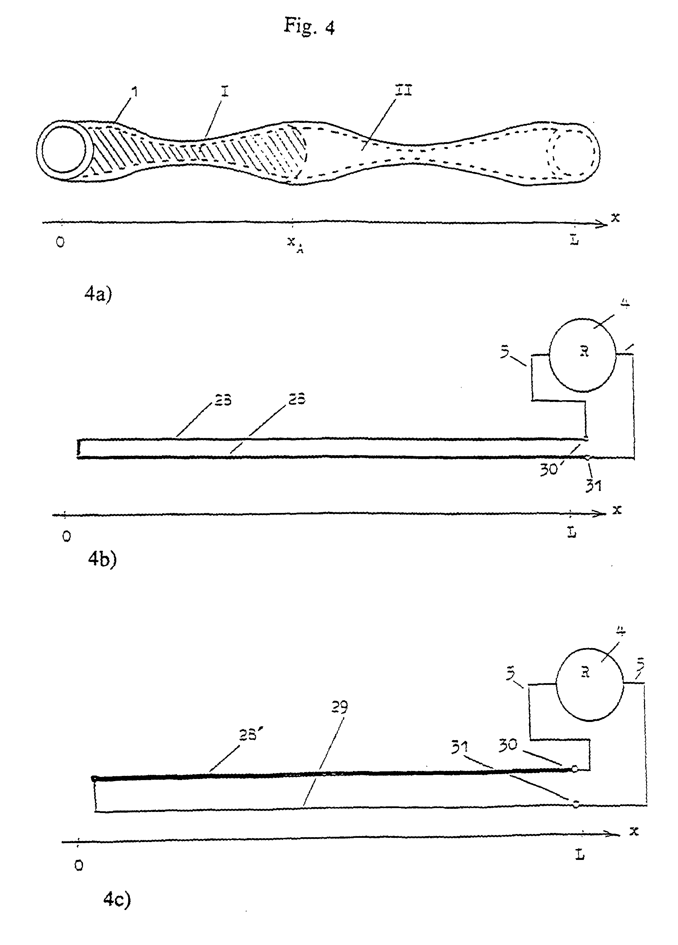

Ein ähnliches Meßprinzip ergibt sich, wenn der Schlauch (1) einen sehr hohen elektrischen Widerstand $R_{so}$ besitzt. In den inneren Hohlraum des Schlauches (1) (Fig. 4a) wird ein elektrischer Leiter (28) (z.B. Widerstandsdraht) mit einem elektrischen Widerstand $R_{sd}$

eingelegt (Fig. 4b + c). Für die Widerstände $R_{so}$ und $R_{sd}$ sollte gelten: $R_{sd} < R_{so}$

Der elektrische Leiter (28) kann z.B. in Form einer Schleife (z.B. Drahtschleife) (Fig. 4b) eingelegt werden.

Bei Befüllung des Schlauches (1) mit einem Stoff (I), der im Vergleich zum elektrischen Leiter (28) einen geringen spezifischen Widerstand besitzt, wird der Widerstandsanteil des elektrischen Leiters (28) zwischen x=0 und x=$x_A$ annähernd kurzgeschlossen. Analog zu dem oben dargestellten Beispiel ergibt sich für den zwischen den Anschlußpunkten (30) und (31) mit dem Widerstandsmeßgerät (4) meßbaren elektrischen Widerstand:

$$\frac{dR}{dV_f} \sim \left( -\frac{1}{A(x_A)} \right)$$

Die Druckprofilmessung kann in der schon beschriebenen Weise erfolgen.

Ebenso ist es möglich, nur einen elektrischen Leiter (28') (z.B. Widerstandsdraht) in den inneren Hohlraum des Schlauches (1) einzubringen. Die Schließung des Stromkreises erfolgt mit Hilfe eines niederohmigen elektrischen Leiters (29) (z.B. Draht) der mit oder ohne äußere elektrische Isolation ausgeführt sein kann.

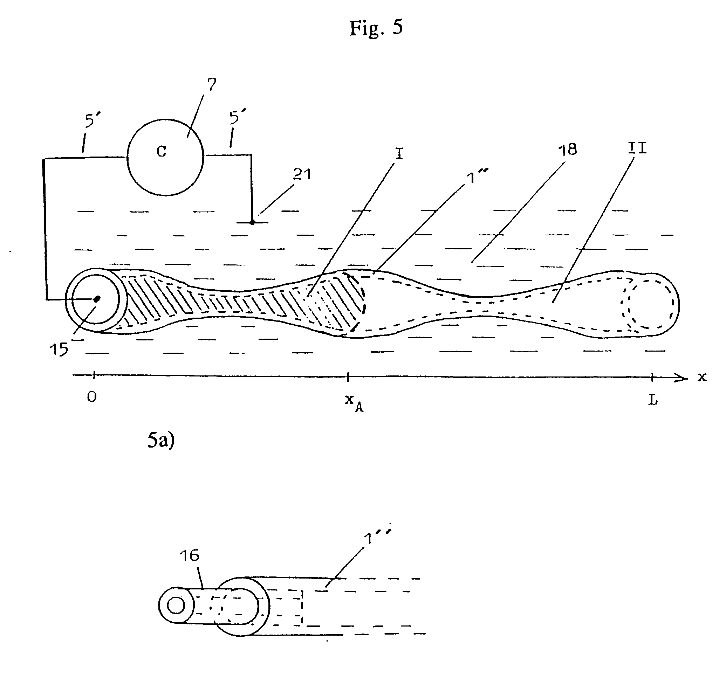

Die Druckprofilmessung läßt sich auch auf der Basis einer Kapazitätsmessung durchführen. Hierfür wird der Schlauch (1") aus einem elektrisch isolierenden Material (Fig. 5a) hergestellt. Die Kapazität kann mit Hilfe eines Kapaziätsmeßgerätes (7) zwischen der elektrisch leitfähigen Füllung (I) und einem elektrisch leitfähigen Medium (18) gemessen werden, das den Schlauch (1") umgibt.

Die meßbare Kapazität C nimmt als Funktion der Füllänge $x_A$ zu.

Figur 5b zeigt die Möglichkeit zur Kontaktierung des Stoffes (I) mit Hilfe eines kurzen Rohres (16), das in das Ende des Schlauches (1") eingesetzt wird.

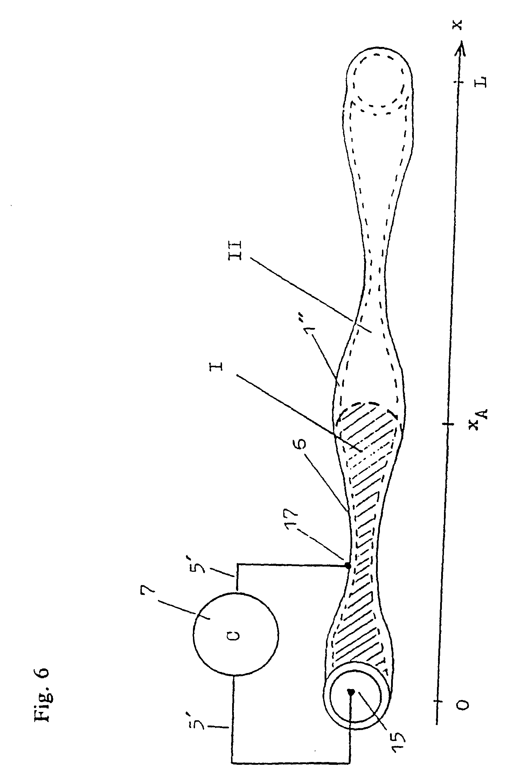

Ebenso ist es möglich, die Kapazitätsmessung zwischen dem elektrisch leitfähigen Stoff (I) und einer elektrisch leitfähigen Beschichtung (6) durchzuführen, die sich auf der Oberfläche des schlauchartigen flexiblen Hohlkörpers (1") befindet (Fig. 6).

Für die meßbare Kapazität C ergibt sich:

$$C = C_f x_A / L$$

Hierin ist $C_f$ die Kapazität des gefüllten Schlauches.

$x_A$ ergibt sich zu

$$x_A = L\ C/C_f$$

Der Differentialquotient $dC/dV_f$ ist das Abbild des Druckprofiles über der Füllänge $x_A$

Die Füllänge $x_A$ kann ebenso auf akustischem Wege erfaßt werden. Wird als Stoff (II) Luft oder ein anderes Gas verwendet, so kann durch akustische Anregung dieser Gassäule die nicht befüllte Länge $(L-x_A)$ des Schlauches (1) durch Bestimmung der Resonanzfrequenz gemessen werden.

Die mit der Erfindung erzielten Vorteile liegen insbesondere darin, daß Druckprofile gemessen werden können, ohne daß der Meßkatheter dabei maschinell bewegt werden muß. Darüber hinaus ist es möglich, durch periodische Vergrößerung, bzw. Verringerung der Füllhöhe $x_A$ bestimmte Bereiche abzuscannen. Durch Variation des Gegendrucks können Informationen über die Festigkeit des druckausübenden Materials gewonnen werden.

Ausführungsbesipiele der Erfindung sind in der Zeichnung dargestellt und werden in der nachfolgenden Beschreibung näher erläutert.

Verschiedene Ausführungsformen der Erfindung sind in den Figuren 3 bis 9 dargestellt. Es zeigen:

Figur 1: a) eine äußere Druckbelastung in Abhängigkeit vom Weg;

b) einen Meßkatheter in Form eines Schlauches unter der Druckbelastung;

c) die Querschnittsfunktion des Meßkatheters bei der Druckbelastung.

Figur 2: einen mit verschiedenen Stoffen gefüllten Meßkatheter.

Figur 3: Meßprinzip auf der Basis einer Widerstandsmessung.

Figur 4: Meßprinzipvariationen auf der Basis einer Widerstandsmessung.

Figur 5: a) Meßprinzip auf der Basis einer Kapazitätsmessung

b) elektrische Kontaktierung des Stoffes (I)

Figur 6: Meßprinzip nach Fig. 5 mit einer zusätzlichen elektrisch leitfähigen Schicht (6).

Figur 7: Doppellumige Ausführung eines Meßkatheters.

Figur 8: Meßkatheter mit unsymmetrischer Wandverstärkung.

Figur 9: Meßkatheter mit 4 um 90° versetzten Meßschläuchen.

Figur 10: eine Ansicht eines weiteren Ausführungsbeispieles eines Schlauches.

Figur 11: ein weiteres Ausführungsbeispiel eines weiteren Meßprinzips.

Figur 12: Anordnungen von Leiterbahnen im Schlauch zur mehrdimensionalen Messung des Druckprofils.

Figur 3 zeigt eine Ausführungsform der Erfindung, die auf der Basis einer elektrischen Widerstandsmessung arbeitet. Der Schlauch (1') ist z.B. aus Silicon, Kautschuk, Latex, Polyurethan, PVC, PP, PE, PA, PUR oder einem anderen Material hergestellt.

Solche Kunststoffe lassen sich durch Dotierung mit Kohlenstoff oder Metallpulver elektrisch leitfähig machen (vgl. Produktinformation unter http:///www prefixfi/preelec.htm) .

Der elektrische Widerstand des Schlauches der Länge L liegt bei $10^6$ Ω. Die Länge L beträgt 1 cm bis 10 m, vorzugsweise 1 m. Der Schlauch hat einen äußeren Durchmesser zwischen 1 mm und 10 cm, vorzugsweise im Bereich von 2 mm bis 5 mm. Die Schlauchwandstärke liegt zwischen 0,1 mm und 5 mm vorzugsweise im Bereich zwischen 0,2 mm und 1 mm.

Ein Widerstandsmeßgerät (4) wird über die elektrischen Zuleitungen (5) mit dem Schlauch (1') verbunden. Die Kontaktierung erfolgt z.B. mit Hilfe von Leitsilber oder Leitkleber an den Punkten (19) und (20).

Mit Hilfe einer Pumpe wird der Schlauch (1') an der Stelle x=0 kontinuierlich mit dem Stoff (I) (z.B. einmolare KCl-Lösung mit einem spezifischen Leitwert von ca. 0,1 S/cm oder NaCl-Lösung...) befüllt und der Widerstand R als Funktion des Füllvolumens $V_f$ gemessen. Das Druckprofil wird wie oben dargestellt ermittelt.

In einem anderen Ausführungsbeispiel (ohne Abbildung) wird der Schlauch (1) aus einem elektrisch isolierenden Material hergestellt. Die innere Schlauchoberfläche ist mit einer elektrisch leitfähigen Schicht überzogen, die wie im vorangegangenen Beispiel über die elektrischen Zuleitungen (5) mit dem Widerstandsmeßgerät (4) verbunden ist.

In einer weiteren Ausführungsform wird in den inneren Hohlraum des elektrisch isolierenden Schlauches (1) eine Widerstanddrahtschleife (Fig. 4b) (z.B. aus Konstantan) mit einem elektrischen Widerstand von $10^6$ Ω eingelegt. Dieser Draht wird über die Anschlußpunkte (30) und (31) und die elektrischen Zuleitungen (5) mit dem Widerstandsmeßgerät (4) verbunden.

In Figur 4c ist der elektrische Leiter (28') mit einem niederohmigen und äußerlich elektrisch isoliertem Leiter (29) (z.B. Draht) verbunden und über die Anschlußpunkte (30) und (31) und die elektrischen Zuleitungen mit dem Widerstandsmeßgerät (4) verbunden.

Es ist ebenso möglich, den elektrischen Leiter (28') spiralförmig um den elektrischen Leiter (29) zu winden (ohne Figur).

Auch können die elektrischen Leiter (28, 28', 29) auf einem beliebigen elektrisch isolierenden und flexiblen Trägermaterial nach dem Stand der Technik in Dickschicht- oder Dünnfilmtechnologie aufgebracht und das flexible Trägermaterial in den inneren Hohlraum des Schlauches (1) eingelegt werden.

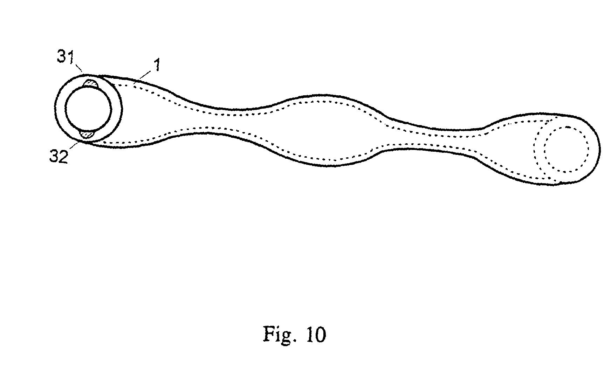

Ein weiteres Ausführungsbeispiel ist in der Fig. 10 dargestellt. Sie zeigt in Anlehnung an Fig. 4 einen elektrisch isolierenden Schlauch (1), der z.B. aus Silikon besteht, und in den durch Koextrusion von elektrisch leitfähigem Silikon zwei Widerstandsbahnen (31, 32) mit gleichen oder unterschiedlichen Widerstandswerten entlang der gesamten Schlauchlänge eingebracht wurden.

Entscheidend ist, daß der Widerstandswert mindestens einer Widerstandsbahn (31 oder 32) pro Längeneinheit erheblich größer ist (z.B. 10 k $\Omega$) als der Widerstandswert des Stoffes I (z.B. 300 $\Omega$) pro Längeneinheit.

Werden die Widerstandsbahnen (31 und 32) am rechten Ende des Schlauches (1) wie in Fig. 4 beschrieben an ein Widerstandsmeßgerät angeschlossen, so können Druckprofile gemessen werden.

Figur 5 zeigt eine weitere Ausführungsform der Erfindung. Die Druckprofilmessung erfolgt hier auf der Basis einer Kapazitätsmessung mit Hilfe eines Kapazitätsmeßgerätes (7). Die elektrische Kapazität wird zwischen dem elektrisch leitenden Stoff (I) und einem elektrisch leitfähigen Medium (18) gemessen, das den Schlauch (1") umgibt. Der Schlauch (1") ist z.B. aus elektrisch isolierendem PU-Material hergestellt, das als Dielektrikum der elektrischen Kapazität wirkt. Das Medium (18) kann eine Kochsalzlösung sein, die mit Hilfe einer Metallelektrode (21) (z.B. aus Gold, Silber oder Kohlenstoff) kontaktiert wird. Die Kontaktierung des Stoffes (I) im Inneren des Schlauches (1) an der Stelle (15) ist in Figur 5a schematisch dargestellt. Eine mögliche Realisierung dieses elektrischen Kontaktes ist in Figur 5b gezeigt. Am Ende des Schlauches (1") ist ein kurzes Metallrohr (16) eingesetzt, das bei Befüllung des Schlauches (1") mit dem Stoff (I) in Kontakt kommt. Das Rohr (16) wird über die elektrische Zuleitung (5') mit dem Kapazitätsmeßgerät (7) verbunden.

Die Messung des Druckprofils geschieht in der oben dargestellten Weise.

Erfolgt die Druckprofilbestimmung auf der Basis einer Kapazitätsmessung nicht in einem elektrisch leitfähigen Medium (18), so kann in einem anderen Ausführungsbeispiel (Fig. 6) die Kapazitätsmessung zwischen Stoff (I) und einer elektrisch leitfähigen Schicht (6) erfolgen, die sich auf der äußeren Oberfläche des elektrisch isolierenden Schlauches (1") befindet.

Diese Schicht (6) besteht z.B. aus Kohlenstoff, Leitlack oder einem dünnen Metallfilm. Sie ist auf der gesamten Oberfläche angebracht oder über der Schlauchlänge streifenförmig ausgeführt.

Solche Filme lassen sich aufsprühen, durch Tauchverfahren oder Bedampfungsverfahren im Vakuum aufbringen.

Die Schicht (6) wird z.B. am Punkt (17) mit Leitsilber mit der elektrischen Zuleitung (5') des Kapazitätsmeßgerätes (7) verbunden.

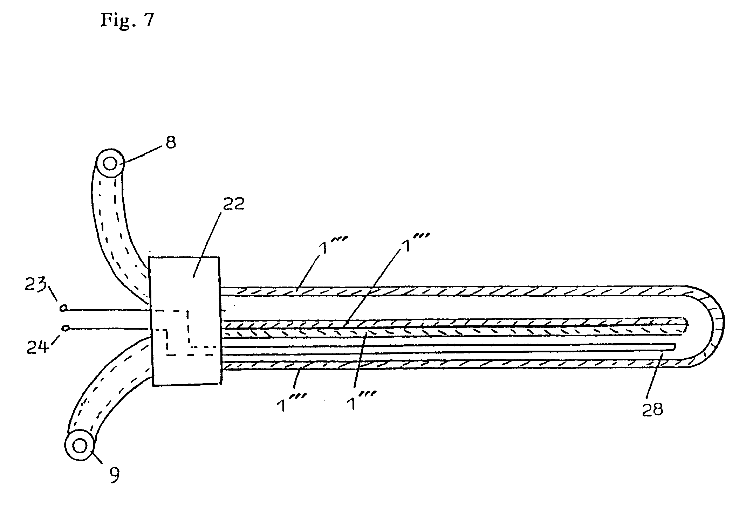

Eine doppellumige Ausführungsform eines Meßkatheters ist in Fig. 7 dargestellt. Hierin ist (1''') der flexible Schlauch (doppellumig). Die Länge des Schlauches zwischen seiner Spitze (rechts in Fig. 7) und dem Anschlußblock (22) beträgt 1 m. Der äußere Durchmesser des doppellumigen Schlauches (1''') beträgt 4 mm, die Wandstärke 0,3 mm. Über den Anschlußblock (22) ist der doppellumige Schlauch (1''') mit den Schläuchen (8) und (9) verbunden, über die Zu- bzw. Abführung der Stoffe (I) und (II) erfolgt.

Die Messung des Meßprofils geschieht analog zu Fig. 4b mit einer Schleife aus Widerstandsdraht (28). Zur Messung wird über die Anschlußpunkte (23) und (24) ein Widerstandsmeßgerät angeschlossen.

Zur Messung des Druckprofils wird als Stoff (I) z.B. einmolare Kochsalzlösung über den Schlauch (8) mit Hilfe einer externen Pumpe zugeführt. Die Luft oder ein anderes Gas kann aus dem Schlauch (1''') über den Schlauch (9) entweichen. An den Schlauch (9) kann ein Drucksensor oder ein regelbares Ventil angeschlossen werden. Mit Hilfe des Sensors und des Ventils ist es möglich, den inneren Druck im Schlauch (1''') zu kontrollieren. Die Differenz der Drücke außerhalb und innerhalb des Schlauches (1''') bestimmt den auswertbaren Druckmeßbereich.

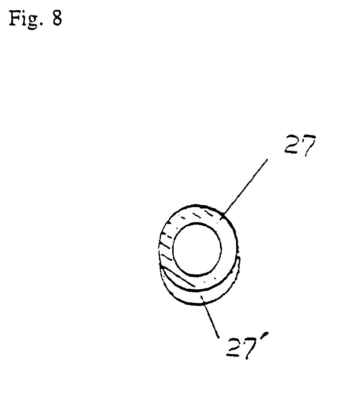

In Fig. 8 ist als Querschnitt ein einlumiger Schlauch zur Druckprofilmessung dargestellt. Der unsymmetrische Aufbau der Wandung ermöglicht eine Querschnittsveränderung auch bei gleichmäßig radial auftretender Druckdifferenz zwischen Innen- und Außendruck. Die Wandungsbereiche (27) und (27') können aus unterschiedlichen Materialien hergestellt sein. Für die Wandung (27) wird vorzugsweise ein flexibles Material und für die Wandung (27') ein festeres Material verwendet.

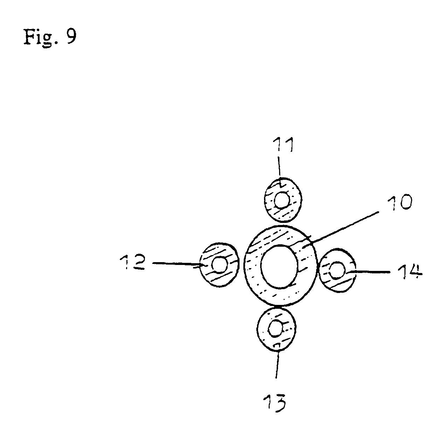

Fig. 9 zeigt einen Meßkatheter mit 4 um 90° versetzten Meßschläuchen. Mit Hilfe der Meßschläuche (11, 12, 13 und 14) wird wie oben dargestellt das Druckprofil gemessen, über den Schlauch (10) erfolgt die Rückführung der Stoffe (I) bzw. (II). In die Meßschläuche (11 bis 14) können Widerstandsdrahtanordnungen gemäß Fig. 4a und b eingeführt werden.

In den vorangegangenen Ausführungsbeispielen wurden Meßanordnungen für die eindimensionale Druckprofilmessung angegeben. Es ist auch möglich, zweidimensionale Druckverteilungen zu messen. Dazu werden mehrere Schläuche (1) parallel angeordnet (ohne Figur). Die Zu- bzw. Abführung der Stoffe (I) und (II) kann dadurch erfolgen, daß alle parallel liegenden Schläuche (1) nacheinander befüllt werden. Sie sind also bezüglich der Befüllung seriell angeordnet.

Jeder der Schläuche (1) kann mit einer Anordnung gemäß Fig. 4b oder c ausgerüstet werden. Die verschiedenen elektrischen Leiter (28) werden über die Anschlußpunkte (30) und (31) einer Meßelektronik zugeführt.

Ein Meßkatheter kann auch zusätzliche Lumen besitzen, die für andere Zwecke frei sind.

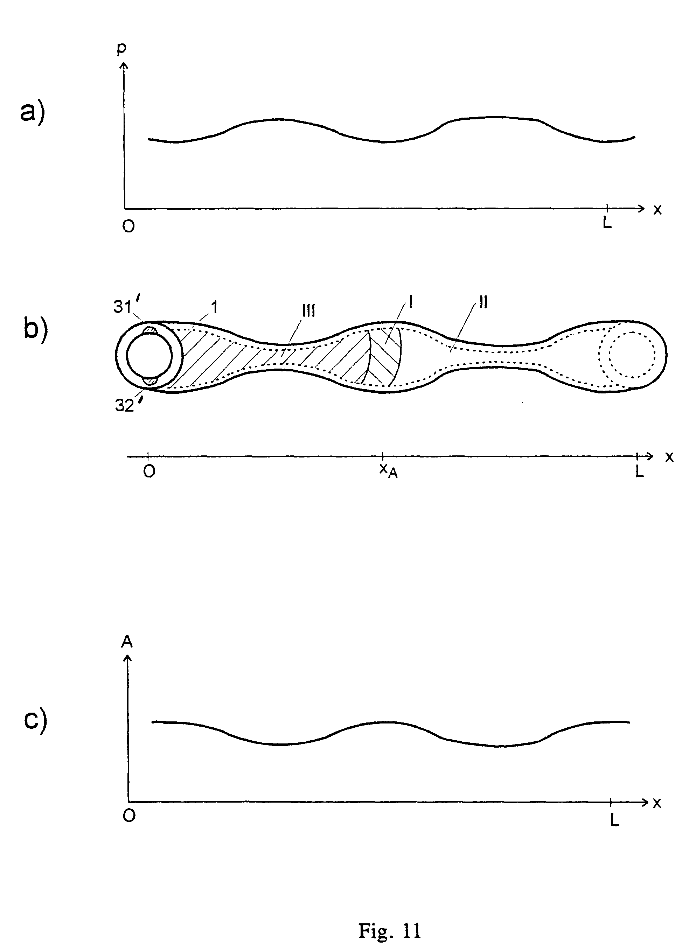

Ein zusätzliches Ausführungsbeispiel der Erfindung zeigt die Fig. 11. Hier sind in einen Silikonschlauch durch Koextrusion zwei Leiterbahnen (31', 32') eingebracht. Diese Leiterbahnen sind möglichst niederohmig zu realisieren. Zur Messung von Druckprofilen wird vom linken Ende des Schlauches (1) ein Stoff I eingefüllt, der nur ein kleines Teilvolumen des Schlauches ausfüllt und den Stoff II verdrängt.

Im Gegensatz zu allen vorhergehenden Ausführungsbeispielen wird nun ein Stoff III in das innere Schlauchvolumen eingebracht. Die Stoffe II und III bestehen z.B. aus Öl, Luft oder jedem anderen flüssigen oder gasförmigen Stoff, der einen sehr hohen spezifischen Widerstand aufweist. Der Stoff I ist dagegen eine Flüssigkeit mit einem geringen spezifischen Widerstand (z.B. Kochsalzlösung).

Eine elektrische Verbindung zwischen den Leiterbahnen (31' und 32') kann so nur über das mit dem Stoff I ausgefüllte Teilvolumen des Schlauches erfolgen. Eine durch einen äußeren Druck bedingte Querschnittsveränderung des Schlauches verursacht eine Verringerung des Abstandes zwischen den Leiterbahnen (31' und 32'). Dieses führt im Bereich des Teilvolumens des Stoffes I zu einer meßbaren Veränderung des elektrischen Widerstandes oder der elektrischen Kapazität.

Bei dieser Ausführungsform kann das Druckprofil abgetastet werden, wie in den vorangegangenen Beispielen. Allerdings muß hier nicht ein Differentialquotient gebildet werden, da die meßbare Widerstandsänderung direkt proportional zur Druckänderung ist.

Zusätzlich ist es bei dieser Ausführungsform möglich, bei konstanter Füllänge $x_A$ den Druck am Ort $x_A$ als Funktion p(t) der Zeit zu messen.

Hier kann ein alternatives Verfahren zur Bestimmung der Füllänge $x_A$ angewendet werden, wenn die Leiterbahnen 31' und 32' unterschiedliche Widerstände je Längeneinheit aufweisen. Der zwischen dem linken Ende der Widerstandsbahn 31' und dem rechten Ende der Widerstandsbahn 32' bzw. dem linken Ende der Widerstandsbahn 32' und dem rechten Ende der Widerstandsbahn 31' meßbare elektrische Widerstand ist abhängig von der Füllänge $x_A$ und kann somit für die Messung verwendet werden.

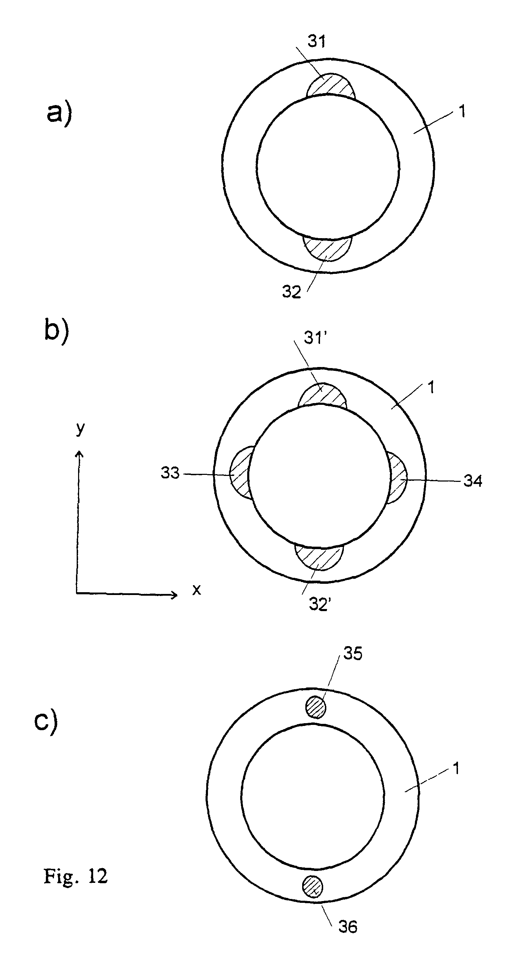

Weitere Ausführungsformen sind in der Fig. 12 dargestellt. Die Fig. 12 zeigt im Querschnitt den schlauchartigen Hohlkörper. In Fig. 12a ist der Schlauch 1 mit den Widerstandsbahnen (31 und 32) gezeigt, wie er im Ausführungsbeispiel nach Fig. 10 beschrieben wurde.

Es ist auch möglich, Druckänderungen am Schlauch (1) nach der X- bzw. Y-Komponente aufzulösen. Hierzu werden zwei Widerstandsbahnen (31' und 32') sowie zwei weitere Widerstandsbahnen (33 und 34) in den selben Schlauch (1) eingebracht. Widerstandsmessungen an dem Widerstandsbahnenpaar (31' und 32') lösen eine Druckkomponente in Y-Richtung auf, während am Widerstandspaar (33 und 34) die X-Komponente gemessen wird. Für kapazitive Messungen sind Leiterbahnen (35 und 36) im Schlauch (1) anwendbar (Fig. 12c). Hierbei kann eine Druckmessung als Funktion des Ortes und als Funktion der Zeit in Anlehnung an die Ausführungsform nach Fig. 11 dadurch erfolgen, daß die Widerstandsmessung durch eine Kapazitätsmessung an den Leiterbahnen (35 und 36) durchgeführt wird. Da die Stoffe II und III einen vernachlässigbaren elektrischen Leitwert besitzen, trägt nur das Teilvolumen des Schlauches (1) das mit dem Stoff I gefüllt ist, zur Kapazitätsmessung bei.

## Patentansprüche

1. Sensorsystem zur Messung von Druckprofilen mit einem Meßkatheter, der als schlauchartiger flexibler Hohlkörper (1) der Länge L ausgebildet ist, und mit einem Stoff (II) gefüllt ist, **dadurch gekennzeichnet,**
**daß** das Sensorsystem Mittel zur kontinuierlichen oder abschnittsweisen Befüllung des mit dem Stoff (II) gefüllten Hohlkörpers von einer Seite her bei x=o mit einem flüssigen Stoff (I) umfaßt, der den Stoff (II) bis zu einer Füllänge $x_A$ verdrängt, wobei der Stoff (II) ein Gas ist oder einen anderen spezifischen Widerstand als der Stoff (I) besitzt, und das Sensorsystem Mittel zur Abtastung einer ortsabhängigen Querschnittsfunktion A(x) des schlauchartigen, flexiblen Hohlkörpers umfaßt, durch die eine äußere Druckbelastung p(x) abgebildet wird.

2. Sensorsystem nach Anspruch 1, **dadurch gekennzeichnet, daß** der Zusammenhang zwischen der äußeren Druckbelastung p(x) und der Querschnittsfunktion A(x) im Sinne einer Meßbereichseinstellung durch eine Veränderung des im Inneren des Schlauches (1) vorgegebenen Drucks $p_o$ variierbar ist.

3. Sensorsystem nach den Ansprüchen 1 und 2, **dadurch gekennzeichnet, daß** das Sensorsystem Mittel zur Erfassung der Füllänge $x_A$ durch Messung des elektrischen Widerstandes und/oder der elektrischen Kapazität umfaßt, wobei der Hohlkörper (1) zunächst in einem kleinen Abschnitt mit dem Stoff (I) befüllbar ist, derart, daß er den Stoff (II) bis zur Füllänge $x_A$ verdrängt, und ferner der Hohlkörper mit einem Stoff (III) befüllbar ist derart, daß er sich im Inneren des Hohlkörpervolumens unmittelbar an Stoff (I) anschließt, wobei die Stoffe (II) und (III) sehr hohe spezifische Widerstände und Stoff (I) einen sehr geringen spezifischen Widerstand aufweisen und das Mittel zur Erfassung der Fülllänge $x_A$ ein Widerstandsmeßgerät und/oder Kapazitätsmeßgerät aufweist, das an Leiterbahnen (31' und 32') angeschlossen ist, die an oder in dem Schlauch angeordnet sind, und das Druckänderungen als Änderungen des elektrischen Widerstandes bzw. der elektrischen Kapazität erfaßt.

4. Sensorsystem nach einem der Ansprüche 1 oder 2, **dadurch gekennzeichnet, daß** das Sensorsystem Mittel zur Erfassung der Fülllänge $x_A$ durch Messung des elektrischen Widerstandes umfaßt, wobei der Hohlkörper (1') einen elektrischen Widerstand im Bereich von $10^2$ bis $10^7$ $\Omega$, der Stoff (I) einen spezifischen Widerstand $\rho 1$ und der Stoff (II) einen spezifischen Widerstand $\rho 2$ mit $\rho 2 \gg \rho 1$ aufweist, und der Verlauf des Druckprofiles p(x) durch den Betrag des Differentialquotienten $dR/dV_f$ über der Fülllänge $x_A$

$$x_A \approx L\left(1 - \frac{R}{R_s}\right)$$

abgebildet wird, wobei $R_s$ der elektrische Widerstand des leeren Hohlkörpers, R der elektrische Widerstand des bis $x_A$ befüllten Hohlkörpers und $V_f$ das Füllvolumen mit Stoff (I) darstellen.

5. Sensorsystem nach einem der vorhergegangenen Ansprüche, **dadurch gekennzeichnet, daß** der Hohlkörper (1') aus elektrisch leitendem Kunststoff hergestellt ist, oder die innere Oberfläche des Hohlkörpers mit einem elektrisch leitenden Film überzogen ist.

6. Sensorsystem nach einem der Ansprüche 1 bis 4, **dadurch gekennzeichnet, daß** der Hohlkörper (1) aus einem elektrisch isolierenden Material besteht und auf der inneren Oberfläche des Hohlkörpers eine elektrisch leitfähige Schicht aufgebracht ist, die über elektrischen Zuleitungen (5) mit einem Widerstandmeßgerät (4) verbunden ist.

7. Sensorsystem nach mindestens einem der Ansprüche 1 bis 4, **dadurch gekennzeichnet, daß** der Hohlkörper aus elektrisch isolierendem Material besteht und. in seinem inneren Hohlraum eine Widerstanddrahtschleife, z.B. aus Konstantan, mit einem elektrischen Widerstand von $10^6$ $\Omega$ eingelegt ist, und dieser Draht über zwei Anschlußpunkte (30) und (31) und elektrischen Zuleitungen (5) mit einem Widerstandsmeßgerät (4) verbunden ist.

8. Sensorsystem nach den Ansprüchen 3 bis 7, **dadurch gekennzeichnet, daß** der Stoff (I) eine elektrisch leitfähige, Flüssigkeit, z.B. Salzlösung: NaCl, KCl..., ist, Stoff (II) und/oder Stoff (III) Luft, ein anderes Gas, oder eine Flüssigkeit, z.B. Öl, ist, wobei der spezifische Widerstand $\rho 2$ des Stoffes II und/oder der spezifische Widerstand $\rho 3$ des Stoffes III mit dem spezifischen Widerstand $\rho 1$ des ersten Stoffes die Bedingung $\rho 2 \gg \rho 1$ und/oder $\rho 3 \gg \rho 1$ erfüllen.

9. Sensorsystem nach einem der Ansprüche 1 bis 3, **dadurch gekennzeichnet, daß** der Hohlkörper (1) einen sehr hohen elektrischen Widerstand $R_{so}$ besitzt, und in den inneren Hohlraum des Hohlkörpers (1) ein elektrischer Leiter (28) mit einem elektrischen Widerstand $R_{sd}$ eingelegt ist, wobei für die Widerstände $R_{so}$ und $R_{sd}$ die Bedingung $R_{sd} < R_{so}$ gilt.

10. Sensorsystem nach Anspruch 9, **dadurch gekennzeichnet, daß** der elektrische Leiter (28) in Form einer Schleife, z.B. einer Drahtschleife, in den inneren Hohlraum des Hohlkörpers eingelegt ist.

11. Sensorsystem nach einem der vorangegangenen Ansprüche, **dadurch gekennzeichnet, daß** ein elektrischer Leiter (28'), z.B. Widerstandsdraht, in dem inneren Hohlraum des Hohlkörpers (1) angeordnet ist und ein niederohmiger elektrischer Leiter (29), z.B. ein Draht, mit oder ohne äußere elektrische Isolation zum Schließen des Stromkreises vorgesehen ist.

12. Sensorsystem nach einem der Ansprüche 1 oder 2, **dadurch gekennzeichnet, daß** mindestens zwei Widerstandsbahnen mit gleichen oder unterschiedlichen Widerstandswerten über die Länge des vorzugsweise elektrisch isolierenden Hohlkörpers an dessen Innenumfang angeordnet sind, wobei an dem dem Mittel zur kontinuierlichen oder abschnittsweisen Befüllung entgegengesetzten Ende des Schlauches ein Widerstandsmeßgerät angeschlossen ist, und daß der Widerstandswert mindestens einer Widerstandsbahn pro Längeneinheit sehr viel größer als der Widerstandswert des Stoffes (I) ist.

13. Sensorsystem nach einem der Ansprüche 1 bis 3, **dadurch gekennzeichnet, daß** der Stoff (I) elektrisch leitfähig ist, der Hohlkörper (1") aus einem elektrisch isolierenden Material hergestellt ist, und das Mittel zur Abtastung einer ortsabhängigen Querschnittsfunktion A(x) ein Mittel zur Bestimmung der Kapazität zwischen dem elektrisch leitfähigen Stoff (I) und einem elektrisch leitfähigen Medium (18), das den Schlauch (1") umgibt, aufweist.

14. Sensorsystem nach einem der Ansprüche 1 bis 3, **dadurch gekennzeichnet, daß** eine elektrisch leitfähige Beschichtung (6) auf der Oberfläche des schlauchartigen flexiblen Hohlkörpers (1") aufgebracht ist, daß der Stoff (I) elektrisch leitfähig ist und das Mittel zur Abtastung einer ortsabhängigen Querschnittsfunktion A(x) ein Mittel zur Bestimmung der Kapazität zwischen der Beschichtung und Stoff (I) aufweist, wobei der Differentialquotient $dC/dV_f$ das Abbild des Druckprofiles über der Füllänge $x_A$ ist, wobei C die bestimmte Kapazität und $V_f$ das Füllvolumen mit Stoff (I) darstellen.

15. Sensorsystem nach dem vorangegangenen Anspruch, **dadurch gekennzeichnet, daß** die elektrisch leitfähige Schicht (6) auf der äußeren Oberfläche des elektrisch isolierenden Hohlkörpers (1") aus Kohlenstoff, Leitlack oder einem dünnen Metallfilm besteht.

16. Sensorsystem nach einem der beiden vorangegangenen Ansprüche, **dadurch gekennzeichnet, daß** die elektrisch leitfähige Schicht (6) auf der äußeren Oberfläche des elektrisch isolierenden Hohlkörpers (1") auf der gesamten Oberfläche des Hohlkörpers angebracht oder über der Länge des Hohlkörpers streifenförmig angeordnet ist.

17. Sensorsystem nach einem der Ansprüche 1 oder 2, **dadurch gekennzeichnet, daß** das Mittel zur Abtastung einer ortabhängigen Querschnittsfunktion A(x) ein Mittel zur Erfassung der Füllänge $x_A$ auf akustischem Wege aufweist, wobei Stoff (II) Luft oder ein anderes Gas ist, und durch akustische Anregung dieser Gassäule die nicht befüllte Länge (L-$x_A$) des Schlauches (1) durch Bestimmung der Resonanzfrequenz bestimmbar ist.

18. Sensorsystem nach einem der vorangegangenen Ansprüche, **dadurch gekennzeichnet, daß** der Hohlkörper (1') Silicon, Kautschuk, Latex, Polyurethan, Polyvinylchlorid (PVC), Polypropylen (PP), Polyethylen (PE), PA, PUR oder ein anderes Material enthält.

19. Sensorsystem nach einem der vorangegangenen Ansprüche, **dadurch gekennzeichnet, daß** der Hohlkörper Kunststoff enthält, der durch Dotierung mit Kohlenstoff oder Metallpulver elektrisch leitfähig gemacht ist, der elektrische Widerstand des Hohlkörpers bei $10^6\,\Omega$ liegt, die Länge L des Hohlkörpers bei 1 cm bis 10 m, vorzugsweise 1 m,liegt, der Hohlkörper einen äußeren Durchmesser zwischen 1 mm und 10 cm, vorzugsweise im Bereich von 2 mm bis 5 mm, aufweist, und/oder die Wandstärke des Hohlkörpers zwischen 0,1 mm und 5 mm, vorzugsweise zwischen 0,2 mm und 1 mm, beträgt.

20. Sensorsystem nach einem der vorangegangenen Ansprüche, **dadurch gekennzeichnet, daß** das Mittel zur kontinuierlichen oder abschnittsweisen Befüllung eine Pumpe zur kontinuierlichen Befüllung des Schlauches (1') an der Stelle x=0 mit dem Stoff (I) aufweist, und daß der Widerstand R als Funktion des Füllvolumens $V_f$ meßbar ist.

21. Sensorsystem nach dem vorangegangenen Anspruch, **dadurch gekennzeichnet, daß** die Widerstandsdrahtschleife einen elektrischen Leiter (28') und einen mit dem elektrischen Leiter (28') elektrisch verbundenen niederohmigen und äußerlich elektrisch isolierten elektrischen Leiter (29), z.B. Draht, aufweist, die über die Anschlußpunkte (30) und (31) und elektrischen Zuleitungen mit dem Widerstandsmeßgerät (4) verbunden sind.

22. Sensorsystem nach dem vorangegangenen Anspruch, **dadurch gekennzeichnet, daß** der elektrische Leiter (28') spiralförmig um den niederohmigen elektrischen Leiter (29) gewunden ist.

23. Sensorsystem nach einem der zwei vorangegangenen Ansprüche, **dadurch gekennzeichnet, daß** die elektrischen Leiter (28, 28', 29) auf einem elektrisch isolierenden und flexiblen Trägermaterial nach dem Stand der Technik in Dickschicht- oder Dünnfilmtechnologie aufgebracht sind, und das flexible Trägermaterial in den inneren Hohlraum des Hohlkörpers (1) eingelegt ist.

24. Sensorsystem nach einem der vorangegangenen Ansprüche, **dadurch gekennzeichnet, daß** der Hohlkörper doppellumig-ist, wobei die beiden Lumina über einen Anschlußblock (22) mit zwei Schläuchen (8 bzw. 9) zur Zu- bzw. Abführung der Stoffe (I) und (II) verbunden sind.

25. Sensorsystem nach einem der vorangegangenen Ansprüche, **dadurch gekennzeichnet, daß** an dem Hohlkörper (9) ein Drucksensor und ein regelbares Ventil angeschlossen ist zur Steuerung des inneren Drucks im Hohlkörper (1''') .

26. Sensorsystem nach einem der vorangegangenen Ansprüche, **dadurch gekennzeichnet, daß** die Wandung des Hohlkörpers unsymmetrisch ist und damit eine Querschnittsveränderung auch bei gleichmäßig radial auftretender Druckdifferenz zwischen Innen und Außendruck erzeugbar ist, wobei die unsymmetrischen Wandungsbereiche (27 bzw. 27') aus unterschiedlichen Materialien bestehen, beispielsweise ein Wandungsbereich (27) aus flexiblem Material und ein anderer Wandungsbereich (27') aus festem Material.

27. Sensorsystem nach einem der vorangegangenen Ansprüche, **dadurch gekennzeichnet, daß** vier Hohlkörper jeweils um 90° versetzt einen Meßkatheter bildend angeordnet sind.

28. Sensorsystem nach dem vorangegangenen Anspruch, **dadurch gekennzeichnet, daß** der Meßkatheter zusätzliche Lumina für andere Zwecke aufweist.

29. Sensorsystem nach einem der vorangegangenen Ansprüche, **dadurch gekennzeichnet, daß** für eine zweidimensionale Messung einer Druckverteilung mehrere Hohlkörper (1) parallel angeordnet sind und die mehreren Hohlkörper zur Zu- bzw. Abführung der Stoffe (I) und (II) nacheinander befüllbar sind.

30. Sensorsystem nach einem der Ansprüche 1 bis 3, **dadurch gekennzeichnet, daß** mindestens zwei Paare von elektrisch leitenden, sich gegenüberliegenden Leiterbahnen an und/oder in dem Hohlkörper angebracht sind und das Mittel zur Abtastung einer ortsabhängigen Querschnittsfunktion A (x) ein Mittel zur Bestimmung des Widerstandes bzw. der Kapazität jeweils eines Paares zur Bestimmung der Druckkomponenten in unterschiedlichen Richtungen aufweist.

31. Sensorsystem nach einem der Ansprüche 1 bis 3, **dadurch gekennzeichnet, daß** eine erste und eine zweite Leiterbahn mit unterschiedlichen Widerständen je Längeneinheit angeordnet sind und der elektrische Widerstand zwischen dem einen Ende der ersten Leiterbahn und dem entgegengesetzten Ende der zweiten Leiterbahn und/oder zwischen dem einen Ende der zweiten Leiterbahn und dem entgegengesetzten Ende der ersten Leiterbahn bestimmbar ist.

32. Sensorsystem nach einem der vorhergegangenen Ansprüche, **dadurch gekennzeichnet, daß** Stoff I eine einmolare KCl-Lösung mit einem spezifischen Leitwert von ca. 0,1 S/cm oder eine NaCl-Lösung ist.

**Claims**

1. A sensor system for measuring pressure profiles, comprising a measuring catheter which is a hose-like, flexible hollow body (1) of length L, and is filled with a substance (II), **characterised in that** the sensor system comprises means for filling, continuously or in sections, the hollow body filled with the substance (II) from one side at x=o with a liquid substance (I) which displaces the substance (II) to a filling length $x_A$, substance (II) being a gas or having a different specific resistance from substance (I), and the sensor system comprising means for sensing a location-dependent cross-sectional function A (x) of the hose-like, flexible hollow body, by means of which an external pressure load p(x) is reproduced.

2. A sensor system according to Claim 1, **characterised in that** the correlation between the external pressure load p(x) and the cross-sectional function A(x) is variable for the purpose of adjusting the measuring region by means of a change of the pressure $p_o$ predetermined in the interior of the hose (1).

3. A sensor system according to Claims 1 and 2, **characterised in that** the sensor system comprises means for detecting the filling length $x_A$ by measuring the electrical resistance and/or the electrical capacitance, wherein the hollow body (1) may be filled first in a small section with the substance (I) in such a way that it displaces the substance (II) to the filling length $x_A$, and furthermore the hollow body may be filled with a substance (III) in such a way that it directly adjoins substance (I) in the interior of the hollow body volume, substances (II) and (III) having very high specific resistances and substance (I) having a very low specific resistance, and the means for detecting the filling length $x_A$ having a resistance-measuring instrument and/or capacitance-measuring instrument which is connected to conductor tracks (31' and 32') disposed on or in the hose, and which detects pressure changes as changes of the electrical resistance or the electrical capacitance.

4. A sensor system according to one of Claims 1 or 2, **characterised in that** the sensor system comprises means for detecting the filling length $x_A$ by measuring the electrical resistance, the hollow body (1') having an electrical resistance in the range of $10^2$ to $10^7$ $\Omega$, substance (I) having a specific resistance $\rho_1$ and substance (II) having a specific resistance $\rho_2$, with $\rho_2 \gg \rho_1$, and the course of the pressure profile p(x) being represented by the amount of the differential quotient $dR/dV_f$ over the filling length $x_A$

$$x_A \approx L\left(1 - \frac{R}{R_s}\right) \; ,$$

$R_s$ representing the electrical resistance of the empty hollow body, R the electrical resistance of the hollow body filled to $x_A$, and $V_f$ the filling volume with substance (I).

5. A sensor system according to one of the preceding Claims, **characterised in that** the hollow body (1') is manufactured from electrically conductive plastics material, or the inner surface of the hollow body is coated with an electrically conductive film.

6. A sensor system according to one of Claims 1 to 4, **characterised in that** the hollow body (1) consists of an electrically insulating material, and an electrically conductive layer is applied on the inner surface of the hollow body, which layer is connected by way of electrical supply lines (5) to a resistance-measuring instrument (4).

7. A sensor system according to at least one of Claims 1 to 4, **characterised in that** the hollow body consists of electrically insulating material and in its inner cavity is inserted a resistance wire loop, made of constantan for example, with an electrical resistance of $10^6 \, \Omega$, and this wire is connected by way of two connection points (30) and (31) and electrical supply lines (5) to a resistance-measuring instrument (4).

8. A sensor system according to Claims 3 to 7, **characterised in that** substance (I) is an electrically conductive liquid, e.g. salt solution: NaCl, KCl ..., substance (II) and/or substance (III) is air, some other gas, or a liquid, e.g. oil, the specific resistance $\rho_2$ of substance II and/or the specific resistance $\rho_3$ of substance III with the specific resistance $\rho_1$ of the first substance fulfilling the condition $\rho_2 >> \rho_1$ and/or $\rho_3 >> \rho_1$.

9. A sensor system according to one of Claims 1 to 3, **characterised in that** the hollow body (1) has a very high electrical resistance $R_{so}$, and an electrical conductor (28) with an electrical resistance $R_{sd}$ is inserted into the inner cavity of the hollow body (1), the condition $R_{sd} < R_{so}$ being valid for the resistances $R_{so}$ and $R_{sd}$.

10. A sensor system according to Claim 9, **characterised in that** the electrical conductor (28) is inserted in the form of a loop, e.g. a wire loop, into the inner cavity of the hollow body.

11. A sensor system according to one of the preceding Claims, **characterised in that** an electrical conductor (28'), e.g. a resistance wire, is disposed in the inner cavity of the hollow body (1), and a low-impedance electrical conductor (29), e.g. a wire, with or without external electrical insulation, is provided to close the circuit.

12. A sensor system according to one of Claims 1 or 2, **characterised in that** at least two resistance tracks with identical or differing resistance values are disposed over the length of the preferably electrically insulating hollow body on the internal periphery thereof, a resistance-measuring instrument being connected to the end of the hose opposite the means for filling continuously or in sections, and **in that** the resistance value of at least one resistance track per unit of length is very much greater than the resistance value of substance (I).

13. A sensor system according to one of Claims 1 to 3, **characterised in that** substance (I) is electrically conductive, the hollow body (1") is manufactured from an electrically insulating material, and the means for sensing a location-dependent cross-sectional function A(x) has means for determining the capacitance between the electrically conductive substance (I) and an electrically conductive medium (18) which surrounds the hose (1").

14. A sensor system according to one of Claims 1 to 3, **characterised in that** an electrically conductive coating (6) is applied on the surface of the hose-like flexible hollow body (1"), **in that** substance (I) is electrically conductive, and the means for sensing a location-dependent cross-sectional function A(x) has means for determining the capacitance between the coating and substance (I), the differential quotient $dC/dV_f$ being the representation of the pressure profile over the filling length $x_A$, C representing the determined capacitance and $V_f$ the filling volume with substance (I).

15. A sensor system according to the preceding Claim, **characterised in that** the electrically conductive layer (6) on the external surface of the electrically insulating hollow body (1") consists of carbon, conductive varnish or a thin metal film.

16. A sensor system according to one of the preceding two Claims, **characterised in that** the electrically conductive layer (6) is applied on the exterior surface of the electrically insulating hollow body (1") on the whole surface of the hollow body or is disposed in strip-form over the length of the hollow body.

17. A sensor system according to one of Claims 1 or 2, **characterised in that** the means for sensing a location-dependent cross-sectional function A(x) has means for detecting the filling length $x_A$ by acoustic means, substance (II) being air or some other gas, and by acoustic excitation of this gas column the non-filled length $(L - x_A)$ of the hose (1) may be determined by determining the resonance frequency.

18. A sensor system according to one of the preceding Claims, **characterised in that** the hollow body (1') contains silicon, rubber, latex, polyurethane, polyvinyl chloride (PVC), polypropylene (PP), polyethylene (PE), PA, PUR or some other material.

**19.** A sensor system according to one of the preceding Claims, **characterised in that** the hollow body contains plastics material which is made electrically conductive by doping with carbon or metal powder, the electrical resistance of the hollow body is $10^6$ $\Omega$, the length L of the hollow body is 1 cm to 10 m, preferably 1 m, the hollow body has an external diameter of between 1 mm and 10 cm, preferably in the range of 2 mm to 5 mm, and/or the wall thickness of the hollow body is between 0.1 mm and 5 mm, preferably between 0.2 mm and 1 mm.

**20.** A sensor system according to one of the preceding Claims, **characterised in that** the means for filling continuously or in sections has a pump for continuously filling the hose (1') at the location x=0 with substance (I), and **in that** the resistance R may be measured as a function of the filling volume $V_f$.

**21.** A sensor system according to the preceding Claim, **characterised in that** the resistance wire loop has an electrical conductor (28') and a low-impedance and externally electrically insulated electrical conductor (29), e.g. wire, electrically connected to the electrical conductor (28'), which conductors are connected by way of the connection points (30) and (31) and electrical supply lines to the resistance-measuring instrument (4).

**22.** A sensor system according to the preceding Claim, **characterised in that** the electrical conductor (28') is wound spirally around the low-impedance electrical conductor (29).

**23.** A sensor system according to one of the preceding two Claims, **characterised in that** the electrical conductors (28, 28', 29) are applied on an electrically insulating and flexible support material according to the prior art, in thick-layer or thin-film technology, and the flexible support material is inserted into the inner cavity of the hollow body (1).

**24.** A sensor system according to one of the preceding Claims, **characterised in that** the hollow body is in double-lumen form, the two lumina being connected by way of a connection block (22) to two hoses (8 or 9) for supplying or removing the substances (I) and (II).

**25.** A sensor system according to one of the preceding Claims, **characterised in that** a pressure sensor and a regulatable valve are connected to the hollow body (9) to control the interior pressure in the hollow body (1''').

**26.** A sensor system according to one of the preceding Claims, **characterised in that** the wall of the hollow body is asymmetrical and thus a change in the cross-section may be produced even when the pressure difference between the internal and external pressure occurs radially evenly, the asymmetrical wall regions (27 or 27') consisting of differing materials, for example one wall region (27) consisting of flexible material and another wall region (27') of rigid material.

**27.** A sensor system according to one of the preceding Claims, **characterised in that** four hollow bodies are disposed so as to be each offset at 90° to one another, forming a measuring catheter.

**28.** A sensor system according to the preceding Claim, **characterised in that** the measuring catheter has additional lumina for other purposes.

**29.** A sensor system according to one of the preceding Claims, **characterised in that** for two-dimensional measurement of pressure distribution, a plurality of hollow bodies (1) are disposed parallel, and the plurality of hollow bodies may be filled in succession in order to supply or remove the substances (I) and (II).

**30.** A sensor system according to one of Claims 1 to 3, **characterised in that** at least two pairs of electrically conductive, mutually opposite conductor tracks are fitted on and/or in the hollow body, and the means for sensing a location-dependent cross-sectional function A (x) has means for determining the resistance or the capacitance of each pair for determining the pressure components in different directions.

**31.** A sensor system according to one of Claims 1 to 3, **characterised in that** a first and a second conductor track are disposed with differing resistances per unit of length, and the electrical resistance between the one end of the first conductor track and the opposite end of the second conductor track and/or between the one end of the second conductor track and the opposite end of the first conductor track may be determined.

**32.** A sensor system according to one of the preceding Claims, **characterised in that** substance I is a one molar KCl solution with a specific conductance of approximately 0.1 S/cm, or a NaCl solution.

**Revendications**

**1.** Système de détection pour la mesure de profils de pression comportant un cathéter de mesure, qui est agencé sous la forme d'un corps creux en forme de tuyau flexible (1) de longueur L et est rempli d'une substance (II), **caractérisé en ce que** le système de détection comprend des moyens pour remplir continûment ou par intervalles le corps creux rempli par la substance (II), à partir d'un côté en x=0 avec une substance liquide (I), qui refoule la substance (II) jusqu'à une longueur de remplissage $x_A$, et dans lequel la substance (II) est un gaz ou possède une résistance spécifique autre que celle de la substance (I) et **en ce que** le système de détection comprend des moyens pour explorer une fonction de section transversale A(x), qui dépend du lieu, du corps creux en forme de tuyau souple, fonction au moyen de laquelle est formée l'image d'une charge de pression extérieure p(x).

2. Système de détection selon la revendication 1, **caractérisé en ce que** la relation entre la charge de pression extérieure p(x) et la fonction de section transversale A(x) peut être modifiée dans le sens d'un réglage de la zone de mesure, moyennant une modification de la pression $p_0$ prédéterminée à l'intérieur du tuyau (1).

3. Système de détection selon les revendications 1 et 2, **caractérisé en ce que** le système de détection comprend des moyens pour détecter la longueur de remplissage $x_A$ par mesure de la résistance électrique et/ou de la capacité électrique, et dans lequel le corps creux (1) peut être rempli tout d'abord dans une petite section, par la substance (I) de telle sorte que cette substance refoule la substance (II) jusqu'à la longueur de remplissage $x_A$, et en outre le corps creux peut être rempli avec une substance (III) de telle sorte qu'elle se raccorde directement à la substance (I) à l'intérieur du volume du corps creux, et dans lequel les substances (II) et (III) possèdent des résistances spécifiques très élevées et la substance (I) possède une résistance spécifique très faible, et **en ce que** le moyen pour détecter la longueur de remplissage $x_A$ possède un appareil de mesure de résistance et/ou un appareil de mesure de capacité, qui est raccordé à des voies conductrices (31' et 32'), qui sont disposées sur ou dans le tuyau et qui détectent des variations de pression en tant que variations de la résistance électrique ou de la capacité électrique.

4. Système de détection selon l'une des revendications 1 ou 2, **caractérisé en ce que** le système de détection comprend des moyens pour détecter la longueur de remplissage $x_A$ par mesure de la résistance électrique, et dans lequel le corps creux (1') possède une résistance électrique dans la gamme comprise entre $10^2$ et $10^7\ \Omega$, **en ce que** la substance (I) possède une résistance spécifique $\rho1$ et que la substance (II) possède une résistance spécifique $\rho2$ avec $\rho2 \gg \rho1$, et la variation du profil de pression p(x) est reproduite au moyen de la valeur du quotient différentiel $dR/dV_F$ sur la longueur de remplissage $x_A$

$$x_A = L\left(1 - \frac{R}{R_s}\right)$$

$R_s$ étant la résistance électrique du corps creux vide, R la résistance électrique du corps creux rempli jusqu'à $x_A$ et $V_f$ le volume de remplissage avec la substance (I).

5. Système de détection selon l'une des revendications précédentes, **caractérisé en ce que** le corps creux (') est réalisé en une matière plastique électriquement conductrice ou la surface intérieure du corps creux est recouverte par un film électriquement conducteur.

6. Système de détection selon l'une des revendications 1 à 4, **caractérisé en ce que** le corps creux () est constitué par un matériau électriquement isolant et **en ce que**, sur la surface intérieure du corps creux, est déposée une couche électriquement conductrice, qui est reliée à l'appareil de mesure de résistance (4) par l'intermédiaire de lignes d'alimentation électrique (5).

7. Système de détection selon au moins l'une des revendications 1 à 4, **caractérisé en ce que** le corps creux est réalisé en un matériel électriquement isolant et qu'une boucle d'un fil résistif, par exemple en constantan ayant une résistance électrique de $10^6\ \Omega$ est insérée dans sa cavité intérieure et que ce fil est relié par l'appareil de mesure de résistance (4) à l'aide de deux points de raccordement (30) et (31) et de lignes d'alimentation électrique (5).

8. Système de détection selon l'une des revendications 3 à 7, **caractérisé en ce que** la substance (I) est un liquide électriquement conducteur, par exemple une solution salée : NaCl, KCl ..., que la substance (II) et/ou la substance (III) sont de l'air, est un autre gaz ou un liquide, par exemple de l'huile, auquel cas la résistance spécifique $\rho_2$ de la substance II et/ou la résistance spécifique $\rho_3$ de la substance III et la résistance spécifique $\rho_1$ de la première substance satisfont à la condition $\rho2 \gg \rho1$ et/ou $\rho3 \gg \rho1$.

9. Système de détection selon l'une des revendications 1 à 3, **caractérisé en ce que** le corps creux (1) possède une résistance électrique $R_{so}$ très élevée et **en ce qu'**un conducteur électrique (28) possédant une résistance électrique $R_{sd}$ est inséré dans la cavité intérieure du corps creux (1), auquel cas on a la condition $R_{sd} < R_{so}$ pour les résistances $R_{so}$ et $R_{sd}$.

**10.** Système de détection selon la revendication 9, **caractérisé en ce que** le conducteur électrique (28) est inséré sous la forme d'une boucle, par exemple d'une boucle de fil, dans la cavité intérieure du corps creux.

**11.** Système de détection selon l'une des revendications précédentes, **caractérisé en ce qu'**un conducteur électrique (28'), par exemple un fil résistif, est disposé dans la cavité intérieure du corps creux (1) et **en ce qu'**un conducteur électrique de faible valeur ohmique (29), par exemple un fil, est prévu avec ou sans isolant électrique extérieure pour fermer le circuit.

**12.** Système de détection selon l'une des revendications 1 ou 2, **caractérisé en ce qu'**au moins deux voies résistives possédant des valeurs résistives identiques ou différentes sont disposées sur la longueur du corps creux de préférence électriquement isolant, sur sa périphérie intérieure, alors qu'un appareil de mesure de résistance est raccordé à l'extrémité du tuyau qui est située à l'opposé du moyen de remplissage continu ou par intervalles, et **en ce que** la valeur résistive est au moins une voie résistive par unité de longueur et nettement plus élevée que la valeur résistive de la substance (I).

**13.** Système de détection selon l'une des revendications 1 à 3, **caractérisé en ce que** la substance (I) est électriquement conductrice, **en ce que** le corps creux (1'') est réalisé en un matériau électriquement isolant, et **en ce que** le moyen d'exploration d'une fonction de section transversale A (x) , qui dépend du lieu, comporte un moyen pour déterminer la capacité entre la substance électriquement conductrice (I) et un milieu électriquement conducteur (18), qui entoure le tuyau (1'').

**14.** Système de détection selon l'une des revendications 1 à 3, **caractérisé en ce qu'**un revêtement électriquement conducteur (6) est déposé sur la surface du corps creux en forme de tuyau flexible (I''), **en ce que** la substance (I) est électriquement conductrice et **en ce que** le milieu pour explorer une fonction transversale A(x), qui dépend du lieu, comporte un milieu pour déterminer la capacité entre le revêtement et la substance (I), le quotient différentiel $dC/dV_f$ représentant l'image du profil de pression sur la longueur de remplissage $x_A$, C étant la capacité déterminée et $V_f$ le volume de remplissage avec la substance (I).

**15.** Système de détection selon l'une des revendications précédentes, **caractérisé en ce que** la couche électriquement conductrice (6) située sur la surface extérieure du corps creux électriquement isolant (1'') est réalisée en carbone, en une laque conductrice ou en un film métallique mince.

**16.** Système de détection selon l'une des revendications précédentes, **caractérisé en ce que** la couche électriquement conductrice (6) est déposée sur la surface extérieure du corps creux électriquement isolant (1'') sur toute la surface du corps creux ou est disposée en forme de bande sur toute la longueur du corps creux.

**17.** Système de détection selon l'une des revendications 1 ou 2, **caractérisé en ce que** le moyen d'exploration d'une fonction de section transversale A (x), qui est fonction du lieu, comporte un moyen pour détecter de façon acoustique la longueur de remplissage $x_A$, la substance (II) étant de l'air ou un autre gaz, et la longueur non remplie $(L-x_A)$ du tuyau (1) pouvant être déterminée au moyen de la détermination de la fréquence de résonance par une excitation acoustique de cette colonne de gaz.

**18.** Système de détection selon l'une des revendications précédentes, **caractérisé en ce que** le corps creux (1') contient du silicone, du caoutchouc, du latex, du polyuréthane, du polychlorure de vinyle (PVC), du polypropylène (PP), du polyéthylène (PE), du PA, du PUR ou un autre matériau.

**19.** Système de détection selon l'une des revendications précédentes, **caractérisé en ce que** le corps creux contient une matière plastique, qui est rendue électriquement conductrice par dopage avec du carbone ou une poudre métallique, **en ce que** la résistance électrique du corps creux est égale à $10^6$ $\Omega$, **en ce que** la longueur L du corps creux est comprise entre 1 cm et 10 m et est de préférence égale à 1 m, **en ce que** le corps creux possède un diamètre extérieur compris entre 1 mm et 10 cm et de préférence dans la gamme comprise entre 2 mm et 5 mm, et/ou **en ce que** l'épaisseur de paroi du corps creux est comprise entre 0,1 mm et 5 mm et de préférence entre 0,2 mm et 1 mm.

**20.** Système de détection selon l'une des revendications précédentes, **caractérisé en ce que** le moyen de remplissage continu ou par intervalles comprend une pompe pour remplir continûment le tuyau (1') au niveau de l'emplacement x=0 avec la substance (I), et **en ce que** la résistance R est mesurable en fonction du volume de remplissage $V_f$.

**21.** Système de détection selon la revendication précédente, **caractérisé en ce que** la boucle de fil résistif possède un conducteur électrique (28') et un conducteur électrique (29), par exemple un fil, de faible valeur ohmique, relié électriquement au conducteur électrique (28') et isolé électriquement extérieurement, ces conducteurs étant reliés à l'appareil de mesure de résistance (4) au moyen des points de raccordement (30) et (31) et de lignes d'alimentation électrique.

**22.** Système de détection selon la revendication précédente, **caractérisé en ce que** le conducteur (28') est enroulé en spirale autour du conducteur électrique (29) de faible valeur ohmique.

**23.** Système de détection selon l'une des deux revendications précédentes, **caractérisé en ce que** les conducteurs électriques (28,28',29) sont déposés sur un matériau de support souple et électriquement isolant, selon l'état de la technique conformément à la technologie des couches épaisses ou des films minces, et **en ce que** le matériau de support souple est inséré dans la cavité intérieure du corps creux (1).

**24.** Système de détection selon l'une des revendications précédentes, **caractérisé en ce que** le corps creux comporte deux lumières, les deux lumières étant reliées par l'intermédiaire d'un bloc de raccordement (22) à deux tuyaux (8 et 9) pour l'amenée et l'évacuation des substances (I) et (II).

**25.** Système de détection selon l'une des revendications précédentes, **caractérisé en ce qu'**un capteur de pression et une soupape réglable sont raccordés au corps creux (9) pour commander la pression intérieure dans le corps creux (1''') .

**26.** Système de détection selon l'une des revendications précédentes, **caractérisé en ce que** la paroi du corps creux est dissymétrique et par conséquent une variation de la section transversale peut être obtenue également pour une différence de pression qui apparaît uniformément du point de vue radial, entre la pression intérieure et la pression extérieure, des parties dissymétriques de paroi (27 et 27') étant réalisées en des matériaux différents, et par exemple une zone de paroi (27) est réalisée en un matériau flexible et une autre zone de paroi (27') est réalisée en un matériau rigide.

**27.** Système de détection selon l'une des revendications précédentes, **caractérisé en ce que** quatre corps creux sont disposés en étant décalés réciproquement de 90° de manière à former un cathéter de mesure.

**28.** Système de détection selon l'une des revendications précédentes, **caractérisé en ce que** le cathéter de mesure comporte des lumières supplémentaires utilisables dans d'autres buts.

**29.** Système de détection selon l'une des revendications précédentes, **caractérisé en ce que** plusieurs corps creux (1) sont disposés en parallèle pour une mesure bidimensionnelle d'une distribution de pression et que la pluralité de corps creux peuvent être chargés successivement pour l'amenée et l'évacuation des substances (I) et (II) .

**30.** Système de détection selon l'une des revendications 1 à 3, **caractérisé en ce qu'**au moins deux paires de voies conductrices, qui sont électriquement conductrices et disposées en vis-à-vis, sont placées sur et/ou dans le corps creux et **en ce que** le moyen d'exploration d'une fonction de section transversale A(x), qui dépend du lieu, comporte un moyen pour déterminer la résistance ou la capacité respectivement d'un couple pour déterminer les composantes de pression dans des directions différentes.

**31.** Système de détection selon l'une des revendications 1 à 3, **caractérisé en ce qu'**il est prévu une première voie conductrice et une seconde voie conductrice possédant des résistances différentes par unité de longueur et que la résistance électrique entre une extrémité de la première voie conductrice et une extrémité opposée de la seconde voie conductrice et/ou entre une extrémité de la seconde voie conductrice et l'extrémité opposée de la première voie conductrice peut être déterminée.

**32.** Système de détection selon l'une des revendications précédentes, **caractérisé en ce que** la substance (I) est une solution unimolaire de KCl possédant une conductivité spécifique d'environ 0,1 S/cm, ou une solution de NaCl.

Fig. 1

1a)

1b)

1c)

Fig. 2

EP 0 986 325 B1

Fig. 3

Fig. 4

4a)

4b)

4c)

Fig. 5

5a)

EP 0 986 325 B1

EP 0 986 325 B1

Fig. 6

EP 0 986 325 B1

Fig. 7

Fig. 8

Fig. 9

Fig. 10

a)

p

O    L    x

b)

31′
32′
1
III
I
II

O    x_A    L    x

c)

A

O    L    x

Fig. 11

Fig. 12